# EUROPÄISCHE PATENTSCHRIFT

(11) **EP 2 670 420 B1**
(45) Veröffentlichungstag und Bekanntmachung des Hinweises auf die Patenterteilung: **27.07.2016**
(21) Anmeldenummer: 12707710.5
(22) Anmeldetag: 01.02.2012
(51) Int. Cl.: A61K 38/16, A61P 31/12, A61K 45/06, C07K 14/42

(54) **ANTIVIRALES MITTEL ENTHALTEND REKOMBINANTE MISTELLEKTINE**
ANTIVIRAL AGENT CONTAINING RECOMBINANT MISTLETOE LECTINS
AGENT ANTIVIRAL CONTENANT DES LECTINES DU GUI RECOMBINANTES

(30) Priorität: 01.02.2011 DE 102011003478
(43) Veröffentlichungstag der Anmeldung: 11.12.2013
(73) Patentinhaber: Cytavis BioPharma GmbH, 22525 Hamburg (DE)
(72) Erfinder: LENTZEN, Hans, 51503 Rösrath (DE); WITTHOHN, Klaus, 51491 Overath (DE)
(74) Vertreter: Simandi, Claus
(86) Internationale Anmeldenummer: PCT/EP2012/051708
(87) Internationale Veröffentlichungsnummer: WO 2012/104355

(56) Entgegenhaltungen:
- EP-A1- 0 751 221
- EP-A2- 1 012 256
- DE-A1- 10 149 030
- DE-A1- 19 804 210
- US-B1- 6 531 125
- LAVELLE ET AL: "Mistletoe lectins enhance immune responses to intranasally co-administered herpes simplex virus glycoprotein D2.", IMMUNOLOGY, Bd. 107, Nr. 2, 1. Oktober 2002 (2002-10-01), Seiten 268-274, XP55029875, ISSN: 0019-2805
- SONG ET AL: "Intranasal immunization with influenza virus and Korean mistletoe lectin C (KML-C) induces heterosubtypic immunity in mice", VACCINE, ELSEVIER LTD, GB, Bd. 25, Nr. 34, 26. Juli 2007 (2007-07-26) , Seiten 6359-6366, XP022169848, ISSN: 0264-410X, DOI: 10.1016/J.VACCINE.2007.06.030
- STIRPE F ET AL: "Ribosome-inactivating proteins: progress and problems", CMLS CELLULAR AND MOLECULAR LIFE SCIENCES, BIRKHÄUSER-VERLAG, BA, Bd. 63, Nr. 16, 24. Juni 2006 (2006-06-24) , Seiten 1850-1866, XP019419202, ISSN: 1420-9071, DOI: 10.1007/S00018-006-6078-7
- ALI KARAGÖZ ET AL: "Antiviral potency of mistletoe (Viscum album ssp. album) extracts against human parain?uenza virus type 2 in Vero cells", PHYTOTHERAPY RESEARCH, Bd. 17, Nr. 5, 1. Mai 2003 (2003-05-01), Seiten 560-562, XP55029851, ISSN: 0951-418X, DOI: 10.1002/ptr.1163 in der Anmeldung erwähnt

### SEQUENCE LISTING

<110> Cytavis Biopharma GmbH
<120> Antivirales Mittel enthaltend rekombinante Mistellektine
<130> CYT24WO
<160> 12
<170> PatentIn version 3.3
<210> 1
   <211> 253
   <212> PRT
   <213> Artificial
<220>
   <223> EP0751221 recombinant Protein
<220>
   <221> misc_feature
   <222> (1)..(1)
   <223> Xaa can be Met or can be deleted
<220>
   <221> misc_feature
   <222> (5)..(5)
   <223> Xaa can be Ile or Leu
<220>
   <221> misc_feature
   <222> (16)..(16)
   <223> Xaa can be Glu or Asp
<400> 1
   Xaa Tyr Glu Arg Xaa Arg Leu Arg Val Thr His Gln Thr Thr Gly Xaa
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Gly
       50                  55                  60
   Asp Ser Ile Thr Ala Ala Ile Asp Val Thr Asn Leu Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Ser Ser Leu Pro Phe
               100                 105                 110
   Asn Gly Ser Tyr Pro Asp Leu Glu Arg Tyr Ala Gly His Arg Asp Gln
   115 120 125
   Ile Pro Leu Gly Ile Asp Gln Leu Ile Gln Ser Val Thr Ala Leu Arg
   130 135 140
   Phe Pro Gly Gly Ser Thr Arg Thr Gln Ala Arg Ser Ile Leu Ile Leu
   145 150 155 160
   Ile Gln Met Ile Ser Glu Ala Ala Arg Phe Asn Pro Ile Leu Trp Arg
   165 170 175
   Ala Arg Gln Tyr Ile Asn Ser Gly Ala Ser Phe Leu Pro Asp Val Tyr
   180 185 190
   Met Leu Glu Leu Glu Thr Ser Trp Gly Gln Gln Ser Thr Gln Val Gln
   195 200 205
   His Ser Thr Asp Gly Val Phe Asn Asn Pro Ile Arg Leu Ala Ile Pro
   210 215 220
   Pro Gly Asn Phe Val Thr Leu Thr Asn Val Arg Asp Val Ile Ala Ser
   225 230 235 240
   Leu Ala Ile Met Leu Phe Val Cys Gly Glu Arg Pro Ser
   245 250
<210> 2
   <211> 256
   <212> PRT
   <213> Artificial
<220>
   <223> EP1051495 recombinant Protein
<220>
   <221> misc_feature
   <222> (1)..(1)
   <223> Xaa can be Met or can be deleted
<220>
   <221> misc_feature
   <222> (16)..(16)
   <223> Xaa can be Asp or Glu
<220>
   <221> misc_feature
   <222> (64)..(64)
   <223> Xaa can be Gly or Gln
<220>
   <221> misc_feature
   <222> (67)..(67)
   <223> Xaa can be Ile or Val
<220>
   <221> misc_feature
   <222> (76)..(76)
   <223> Xaa can be Leu or Ala
<220>
   <221> misc_feature
   <222> (108)..(108)
   <223> Xaa can be Asp-Arg or can be deleted
<220>
   <221> misc_feature
   <222> (114)..(114)
   <223> Xaa can be Asn or Thr
<220>
   <221> misc_feature
   <222> (118)..(118)
   <223> Xaa can be Pro or Thr
<220>
   <221> misc_feature
   <222> (135)..(135)
   <223> Xaa can be Asp or Glu
<220>
   <221> misc_feature
   <222> (142)..(142)
   <223> Xaa can be Ser or Thr
<220>
   <221> misc_feature
   <222> (146)..(146)
   <223> Xaa can be Phe or Tyr
<220>
   <221> misc_feature
   <222> (153)..(153)
   <223> Xaa can be Ala or Thr
<220>
   <221> misc_feature
   <222> (178)..(178)
   <223> Xaa can be Ala or Tyr
<220>
   <221> misc_feature
   <222> (181)..(181)
   <223> Xaa can be Tyr or Asp
<220>
   <221> misc_feature
   <222> (186)..(186)
   <223> Xaa can be Ala or Glu
<220>
   <221> misc_feature
   <222> (192)..(192)
   <223> Xaa can be Val or Met
<220>
   <221> misc_feature
   <222> (220)..(220)
   <223> Xaa can be Ile or Phe
<220>
   <221> misc_feature
   <222> (225)..(226)
   <223> Xaa can be Pro-Ser or Pro-Thr
<220>
   <221> misc_feature
   <222> (233)..(233)
   <223> Xaa can be Thr or Ser
<220>
   <221> misc_feature
   <222> (237)..(237)
   <223> Xaa can be Asp or Ser
<220>
   <221> misc_feature
   <222> (255) .. (256)
   <223> Xaa can be Ser-Ser or can be deleted
<400> 2
   Xaa Tyr Glu Arg Leu Arg Leu Arg Val Thr His Gln Thr Thr Gly Xaa
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Xaa
       50                  55                  60
   Asp Ser Xaa Thr Ala Ala Ile Asp Val Thr Asn Xaa Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Xaa Ser Ser Leu Pro
               100                 105                 110
   Phe Xaa Gly Ser Tyr Xaa Asp Leu Glu Arg Tyr Ala Gly His Arg Asp
           115                 120                 125
   Gln Ile Pro Leu Gly Ile Xaa Gln Leu Ile Gln Ser Val Xaa Ala Leu
       130                 135                 140
   Arg Xaa Pro Gly Gly Ser Thr Arg Xaa Gln Ala Arg Ser Ile Leu Ile
   145                 150                 155                 160
   Leu Ile Gln Met Ile Ser Glu Ala Ala Arg Phe Asn Pro Ile Leu Trp
                   165                 170                 175
   Arg Xaa Arg Gln Xaa Ile Asn Ser Gly Xaa Ser Phe Leu Pro Asp Xaa
               180                 185                 190
   Tyr Met Leu Glu Leu Glu Thr Ser Trp Gly Gln Gln Ser Thr Gln Val
           195                 200                 205
   Gln His Ser Thr Asp Gly Val Phe Asn Asn Pro Xaa Arg Leu Ala Ile
       210                 215                 220
   Xaa Xaa Gly Asn Phe Val Thr Leu Xaa Asn Val Arg Xaa Val Ile Ala
   225                 230                 235                 240
   Ser Leu Ala Ile Met Leu Phe Val Cys Gly Glu Arg Pro Ser Xaa Xaa
                   245                 250                 255
<210> 3
   <211> 257
   <212> PRT
   <213> Artificial
<220>
   <223> EP1051495 recombinant Protein
<220>
   <221> misc_feature
   <222> (1)..(1)
   <223> Xaa can be Met or can be deleted
<400> 3
   Xaa Tyr Glu Arg Leu Arg Leu Arg Val Thr His Gln Thr Thr Gly Asp
   1               5                   10                  15
   Glu Tyr Phe Arg Phe Ile Thr Leu Leu Arg Asp Tyr Val Ser Ser Gly
               20                  25                  30
   Ser Phe Ser Asn Glu Ile Pro Leu Leu Arg Gln Ser Thr Ile Pro Val
           35                  40                  45
   Ser Asp Ala Gln Arg Phe Val Leu Val Glu Leu Thr Asn Gln Gly Gln
       50                  55                  60
   Asp Ser Ile Thr Ala Ala Ile Asp Val Thr Asn Ala Tyr Val Val Ala
   65                  70                  75                  80
   Tyr Gln Ala Gly Asp Gln Ser Tyr Phe Leu Arg Asp Ala Pro Arg Gly
                   85                  90                  95
   Ala Glu Thr His Leu Phe Thr Gly Thr Thr Arg Asp Arg Ser Ser Leu
               100                 105                 110
   Pro Phe Thr Gly Ser Tyr Thr Asp Leu Glu Arg Tyr Ala Gly His Arg
           115                 120                 125
   Asp Gln Ile Pro Leu Gly Ile Glu Gln Leu Ile Gln Ser Val Ser Ala
       130                 135                 140
   Leu Arg Tyr Pro Gly Gly Ser Thr Arg Ala Gln Ala Arg Ser Ile Leu
   145                 150                 155                 160
   Ile Leu Ile Gln Met Ile Ser Glu Ala Ala Arg Phe Asn Pro Ile Leu
                   165                 170                 175
   Trp Arg Tyr Arg Gln Asp Ile Asn Ser Gly Glu Ser Phe Leu Pro Asp
               180                 185                 190
   Met Tyr Met Leu Glu Leu Glu Thr Ser Trp Gly Gln Gln Ser Thr Gln
           195                 200                 205
   Val Gln His Ser Thr Asp Gly Val Phe Asn Asn Pro Phe Arg Leu Ala
       210                 215                 220
   Ile Ser Thr Gly Asn Phe Val Thr Leu Ser Asn Val Arg Ser Val Ile
   225                 230                 235                 240
   Ala Ser Leu Ala Ile Met Leu Phe Val Cys Gly Glu Arg Pro Ser Ser
                   245                 250                 255
   Ser
<210> 4
   <211> 264
   <212> PRT
   <213> Artificial
<220>
   <223> EP0751221 recombinant Protein
<220>
   <221> misc_feature
   <222> (1)..(1)
   <223> Xaa can be Met or can be deleted
<400> 4
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe Arg Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Leu Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Lys Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro
               260
<210> 5
   <211> 268
   <212> PRT
   <213> Artificial
<220>
   <223> EP0751221 recombinant Protein
<220>
   <221> misc_feature
   <222> (1)..(1)
   <223> Xaa can be Met or can be deleted
<400> 5
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe Arg Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Leu Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Lys Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro Gly Gly Tyr His
               260                 265
<210> 6
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<220>
   <221> misc_feature
   <222> (19)..(19)
   <223> Xaa can be Asn or Ser
<220>
   <221> misc_feature
   <222> (22)..(22)
   <223> Xaa can be Cys or Arg
<220>
   <221> misc_feature
   <222> (57)..(57)
   <223> Xaa can be Gly or Asn
<220>
   <221> misc_feature
   <222> (96)..(96)
   <223> Xaa can be Gly or Asn
<220>
   <221> misc_feature
   <222> (158)..(158)
   <223> Xaa can be Gly or Gln
<220>
   <221> misc_feature
   <222> (167)..(167)
   <223> Xaa can be Val or Asp
<220>
   <221> misc_feature
   <222> (171)..(171)
   <223> Xaa can be Gln or Lys
<220>
   <221> misc_feature
   <222> (174)..(175)
   <223> Xaa can be Gly or can be deleted or can be Gly-Arg or Gly-Lys or Arg
   or Lys
<220>
   <221> misc_feature
   <222> (196)..(196)
   <223> Xaa can be Cys or Val or Ser
<220>
   <221> misc_feature
   <222> (212)..(213)
   <223> Xaa can be Ala-Ala or Ala-Gly or Gly-Ala or Gly-Gly
<220>
   <221> misc_feature
   <222> (215) .. (216)
   <223> Xaa can be Ser-Ser or Ser-Gly or Gly-Ser or Gly-Gly
<220>
   <221> misc_feature
   <222> (225)..(225)
   <223> Xaa can be Gly or Tyr
<220>
   <221> misc_feature
   <222> (232)..(236)
   <223> Xaa232 can be Asn, Ser, Thr or Lys, Xaa233 can be Ser or Gly, Xaa234
   can be Leu or Pro, Xaa235 can be Ala or Met, Xaa 236 can be Met or Val
<220>
   <221> misc_feature
   <222> (265)..(265)
   <223> Xaa can be Pro or Phe
<400> 6
   Xaa Asp Asp Val Thr Cys Ser Ala Ser Glu Pro Thr Val Arg Ile Val
   1               5                   10                  15
   Gly Arg Xaa Gly Met Xaa Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Xaa Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Xaa
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Xaa Gly Ser
   145                 150                 155                 160
   Val Trp Val Glu Thr Cys Xaa Ser Ser Gln Xaa Asn Gln Xaa Xaa Trp
                   165                 170                 175
   Ala Leu Tyr Gly Asp Gly Ser Ile Arg Pro Lys Gln Asn Gln Asp Gln
               180                 185                 190
   Cys Leu Thr Xaa Gly Arg Asp Ser Val Ser Thr Val Ile Asn Ile Val
           195                 200                 205
   Ser Cys Ser Xaa Xaa Ser Xaa Xaa Gln Arg Trp Val Phe Thr Asn Glu
       210                 215                 220
   Xaa Ala Ile Leu Asn Leu Lys Xaa Xaa Xaa Xaa Xaa Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Xaa
               260                 265
<210> 7
   <211> 264
   <212> PRT
   <213> Artificial
<220>
   <223> EP1051495 recombinant Protein
<220>
   <221> misc_feature
   <222> (1)..(1)
   <223> Xaa can be Met or can be deleted
<400> 7
   Xaa Asp Asp Val Thr Cys Ser Ala Ser Glu Pro Thr Val Arg Ile Val
   1               5                   10                  15
   Gly Arg Asn Gly Met Cys Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Gly
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Val Ser Ser Gln Gln Asn Gln Arg Trp Ala
                   165                 170                 175
   Leu Tyr Gly Asp Gly Ser Ile Arg Pro Lys Gln Asn Gln Asp Gln Cys
               180                 185                 190
   Leu Thr Cys Gly Arg Asp Ser Val Ser Thr Val Ile Asn Ile Val Ser
           195                 200                 205
   Cys Ser Ala Gly Ser Ser Gly Gln Arg Trp Val Phe Thr Asn Glu Gly
       210                 215                 220
   Ala Ile Leu Asn Leu Lys Asn Gly Leu Ala Met Asp Val Ala Gln Ala
   225                 230                 235                 240
   Asn Pro Lys Leu Arg Arg Ile Ile Ile Tyr Pro Ala Thr Gly Lys Pro
                   245                 250                 255
   Asn Gln Met Trp Leu Pro Val Pro
               260
<210> 8
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 8
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Asn Gly Leu Ala Met Asp Val Ala Gln
   225                 230                 235                 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 9
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 9
   Xaa Asp Asp Val Thr Cys Ser Ala Ser Glu Pro Thr Val Arg Ile Val
   1               5                   10                  15
   Gly Arg Ser Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Asn Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gln Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
   180 185 190
   Cys Leu Thr Val Gly Arg Asp Ser Val Ser Thr Val Ile Asn Ile Val
   195 200 205
   Ser Cys Ser Gly Ala Ser Gly Ser Gln Arg Trp Val Phe Thr Asn Glu
   210 215 220
   Tyr Ala Ile Leu Asn Leu Lys Ser Gly Leu Ala Met Asp Val Ala Gln
   225 230 235 240
   Ala Asn Pro Lys Leu Arg Arg Ile Ile Ile Tyr Pro Ala Thr Gly Lys
   245 250 255
   Pro Asn Gln Met Trp Leu Pro Val Phe
   260 265
<210> 10
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 10
   Xaa Asp Asp Val Thr Cys Ser Ala Ser Glu Pro Thr Val Arg Ile Val
   1 5 10 15
   Gly Arg Asn Gly Met Arg Val Asp Val Arg Asp Asp Asp Phe His Asp
   20 25 30
   Gly Asn Gln Ile Gln Leu Trp Pro Ser Lys Ser Asn Asn Asp Pro Asn
   35 40 45
   Gln Leu Trp Thr Ile Lys Arg Asp Gly Thr Ile Arg Ser Asn Gly Ser
   50 55 60
   Cys Leu Thr Thr Tyr Gly Tyr Thr Ala Gly Val Tyr Val Met Ile Phe
   65 70 75 80
   Asp Cys Asn Thr Ala Val Arg Glu Ala Thr Ile Trp Gln Ile Trp Asp
   85 90 95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Thr Gly Leu Ala Met Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265
<210> 11
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 11
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Lys Gly Pro Ala Met Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265
<210> 12
   <211> 265
   <212> PRT
   <213> Artificial
<220>
   <223> EP1051495 recombinant Protein
<220>
   <221> misc_feature
   <222> (1)..(1)
   <223> Xaa can be Met or can be deleted
<400> 12
   Xaa Asp Asp Val Thr Cys Ser Ala Ser Glu Pro Thr Val Arg Ile Val
   1               5                   10                  15
   Gly Arg Asn Gly Met Arg Val Asp Val Arg Asp Asp Asp Phe His Asp
               20                  25                  30
   Gly Asn Gln Ile Gln Leu Trp Pro Ser Lys Ser Asn Asn Asp Pro Asn
           35                  40                  45
   Gln Leu Trp Thr Ile Lys Arg Asp Gly Thr Ile Arg Ser Asn Gly Ser
       50                  55                  60
   Cys Leu Thr Thr Tyr Gly Tyr Thr Ala Gly Val Tyr Val Met Ile Phe
   65                  70                  75                  80
   Asp Cys Asn Thr Ala Val Arg Glu Ala Thr Ile Trp Gln Ile Trp Asp
                   85                  90                  95
   Asn Gly Thr Ile Ile Asn Pro Arg Ser Asn Leu Val Leu Ala Ala Ser
               100                 105                 110
   Ser Gly Ile Lys Gly Thr Thr Leu Thr Val Gln Thr Leu Asp Tyr Thr
           115                 120                 125
   Leu Gly Gln Gly Trp Leu Ala Gly Asn Asp Thr Ala Pro Arg Glu Val
       130                 135                 140
   Thr Ile Tyr Gly Phe Arg Asp Leu Cys Met Glu Ser Asn Gly Gly Ser
   145                 150                 155                 160
   Val Trp Val Glu Thr Cys Asp Ser Ser Gln Lys Asn Gln Gly Lys Trp
                   165                 170                 175
   Ala Leu Tyr Gly Asp Gly Ser Ile Arg Pro Lys Gln Asn Gln Asp Gln
               180                 185                 190
   Cys Leu Thr Ser Gly Arg Asp Ser Val Ser Thr Val Ile Asn Ile Val
           195                 200                 205
   Ser Cys Ser Gly Ala Ser Gly Ser Gln Arg Trp Val Phe Thr Asn Glu
       210                 215                 220
   Gly Ala Ile Leu Asn Leu Lys Asn Ser Leu Met Val Asp Val Ala Gln
   225                 230                 235                 240
   Ala Asn Pro Lys Leu Arg Arg Ile Ile Ile Tyr Pro Ala Thr Gly Lys
                   245                 250                 255
   Pro Asn Gln Met Trp Leu Pro Val Phe
               260                 265

## Beschreibung

Die Erfindung betrifft ein antivirales Mittel enthaltend rekombinante Mistellektine zur Behandlung von Virusinfektionen, sowie ein Arzneimittel und / oder pharmazeutische Zusammensetzung zur Behandlung von Virusinfektionen.

Bei einer Virusinfektion dringen Viren in einen Organismus ein und vermehren sich dort. Die in der Regel darauf folgende Reaktion des Organismus kann sich in einer Infektionskrankheit äußern. Beim Menschen können eine Vielzahl von Krankheiten durch Viren verursacht werden. Nur gegen eine begrenzte Anzahl von Virusinfektionen sind gegenwärtig zur Vorbeuge schützende Impfungen möglich. Da Viren im Gegensatz zu Bakterien keine Zellen sind, können sie auch nicht wie solche abgetötet werden. Es ist lediglich möglich, eine virale Infektion und die Virusvermehrung zu behindern oder zu verhindern.
Im Folgenden wird beispielhaft für die Vielzahl von möglichen Viruserkrankungen eine Infektion mit Herpes-simplex-Viren näher erläutert.

Herpes-simplex-Viren sind weltweit verbreitet und der Mensch ist für sie als Reservoir der einzige natürliche Wirt. In Deutschland konnten bei 84 bis 92% der Personen einer altersnormalisierten Stichprobenuntersuchung Antikörper gegen Herpes-simplex-Viren nachgewiesen werden. Durch das Herpes-simplex-Virus verursachte Erkrankungen, gehören zu den häufigsten Infektionskrankheiten der Haut. Die meisten Infektionen treten im Gesicht und im Genitalbereich auf. Es ist allgemein bekannt, dass das Herpes-simplex-Virus eine rekurrente Virusinfektion ist, welche durch das Erscheinungsbild entweder von einzelnen oder mehreren Ansammlungen von kleinen Vesikeln auf der Haut oder Schleimhautmembranen gekennzeichnet ist.

Es gibt zwei primäre Typen des Herpes-simplex-Virus (HSV) - HSV 1 und HSV 2. Sie unterscheiden sich hinsichtlich ihrer Krankheitsbilder und der Krankheitslokalisation geringfügig. Nach dem Auftreten und der Lokalisation der Krankheitssymptome unterscheidet man klinisch verschiedene HSV-Infektionen, deren wichtigste der *Herpes simplex labialis* (Lippenherpes) und der *Herpes simplex genitalis* darstellen. Nach einer Erstinfektion verbleibt das Virus in einem Ruhezustand (Latenz) stets lebenslang im Organismus, was man als persistierende Infektion bezeichnet. Die Therapie von HSV-Infektionen vermag diese Persistenz nicht zu beenden, sondern sie versucht die Vermehrung des Virus nach einer erfolgten Reaktivierung aus dem Ruhestadium zu verhindern.

Zur Behandlung von Virusinfektionen werden u.a. Wirkstoffe angewandt, die unter der Bezeichnung Virostatika subsummiert werden. Diese Arzneimittel greifen an unterschiedlichen Stellen in die Virusvermehrung ein und verhindern so ein weiteres Ausbreiten des Erregers. Die zur Bekämpfung von Herpes simplex-Viren bei Lippenherpes häufig angewandten Virostatika gehören zur Gruppe der sog. Nukleosidanaloga. Sie hemmen die DNA-Synthese und damit die Virusvermehrung. Da Nukleosidanaloga immer in die Virusvermehrung eingreifen, funktionieren sie nur bei sich aktiv reproduzierenden Viren. Unter diesen Nukleosidanaloga hat sich die als Aciclovir bekannte Verbindung als wirksam erwiesen, die jedoch auch einige Nebenwirkungen aufweist. Die Ausscheidung von Aciclovir findet über die Niere statt. Es sind Nierenprobleme bekannt geworden bei großen, schnell und intravenös verabreichten Dosen, weil dann Aciclovir in den Nieren auskristallisieren kann. Ferner kann Aciclovir auch in die zelluläre DNA eingebaut werden und stellt somit ein chromosomales Mutagen dar. Außerdem können sich Resistenzen entwickeln.
Obwohl die Nukleosidanaloga einen großen Wert besitzen, bleibt das Bedürfnis an verbesserten Medikationen bestehen, um die Symptome, die durch die Ausbrüche von Virusinfektionen auftreten, besser behandeln zu können.
Ferner gibt es einige Viren, die die Entstehung von Krebs begünstigen, wenn sie über lange Zeit eine latente Infektion verursachen. So wurden beispielsweise Papillomviren mit dem Zervixkarzinom in Verbindung gebracht, Epstein-Barr-Virus mit dem Nasopharynxkarzinom.

Mistelextrakte werden seit Jahrhunderten therapeutisch genutzt. Insbesondere in der Krebstherapie sind Mistelpräparate mit unterschiedlichem Erfolg eingesetzt worden (Bocci V 1993 J Biol Regulators and Homeostatic Agents 7(1): 1 - 6; Gabius H-J, Gabius S, Joshi S S et al. 1993 Planta Med 60: 2 - 7; Gabius H-J & Gabius S 1994 PZ 139: 9 - 16; Ganguly C & Das S 1994 Chemotherapy 40: 272 - 278, Hajto T, Hostanska K, Gabius H_J 1989 Cancer Res 49: 4803 - 4808, Hajto T, Hostanska K, Frei K et al. 1990 Cancer Res. 50: 3322 - 3326). Es zeigte sich, dass die therapeutischen Effekte insbesondere durch sogenannte Mistellektine (Viscumine, Viscum album Agglutinine, VAA) vermittelt werden. Den Mistellektinen wird dabei neben einer zytotoxischen Wirkung auch eine unspezifische Immunstimulation zugesprochen, deren positive Effekte zur Therapie von Tumorpatienten genutzt werden. Verschiedene Untersuchungen mit Mistellektinen in vitro (Hajto et al., 1990 (supra); Männel D N, Becker H, Gundt A et al. 1991 Cancer Immunol Immunother 33: 177 - 182; Beuth J, Ko K L, Tunggal L et al. 1993 Drug Res 43: 166 - 169) und in vivo (Hajto T 1986 Oncology 43 suppl 1: 51 - 65; Hajto et al., 1989 (supra), Beuth J, Ko H L, Gabius H-J et al. 1991 In Vivo 5: 29 - 32; Beuth J, Ko H L, Gabius H-J et al. 1992 J Clin Invest 70: 658 - 661), sowie klinische Studien (Beuth et al., 1992 (supra)) zeigten eine erhöhte Freisetzung von inflammmatorischen Zytokinen (TNF-alpha, IL-1, IL-6) und eine Aktivierung von zellulären Komponenten des Immunsystems (TH -Zellen, NK-Zellen).

Nur wenige Studien haben bisher die antivirale Wirksamkeit von nativen Mistellektinen untersucht, die jedoch Extrakte aus Mistellektinen darstellen. Karagöz et al. (Phytother. Res. 17, 560-562, 2003) haben bisher die antivirale Wirksamkeit von Extrakten der Europäischen Mistel (Viscum album L.) untersucht. Karagöz et al erforschten, ob verschiedene Mistelextrakte eine antivirale Wirksamkeit auf das humane Parainfluenzavirus Typ 2 (HPIV-2) aufweisen. Dabei wurden die folgenden Mistellextrakte untersucht: ein wässriger Extrakt, ein Ethanol-Extrakt, ein Petroleum-Ether-Extrakt, ein Chloroform-Extrakt und ein Aceton-Extrakt. Das Testsystem bestand aus Vero-Zellen, dem humanen HPIV-2 und den verschiedenen Mistellextrakten. Es wurden Zytotoxititätstests und Plaque-Assays durchgeführt. Es zeigte sich, dass der wässrige Extrakt eine signifikante Wirkung gegen die HPIV-2-Replikation aufwies, während der Chloroform-Extrakt eine moderate Aktivität besaß. Die restlichen Extrakte zeigten keine signifikante Wirkung auf die HPIV-2-Replikation.
Bei den im Stand der Technik beschriebenen Mistelextrakten handelt es sich um Vielstoffgemische pflanzlichen Ursprungs, deren Inhaltsstoffe nicht beschrieben und auch nicht charakterisiert sind. Somit bleibt mit den Untersuchungen von Karagöz et al. ungeklärt, welche Substanzen in dem wässrigen Extrakt die Aktivität gegen die HPIV-2-Replikation aufwiesen. Pflanzliche Extrakte sind heterogen in der Zusammensetzung ihrer Inhaltsstoffe. Aus diesem Grund bestehen Schwierigkeiten Extrakte hinsichtlich einer pharmakologischen Wirkung auf bestimmte Konzentrationen eines oder mehrerer Inhaltsstoffe einzustellen. Durch Analysen des Mistelextraktes konnten bisher drei Mistellektine (ML-I, ML-II, ML-III) mit unterschiedlichen Molekulargewichten und Zuckerbindungsspezifitäten identifiziert werden. Es konnte gezeigt werden, dass der immunstimulierende Effekt des Mistelextrakts auf das ML-I zurückzuführen ist. Das ML-I-Lektin besteht aus jeweils zwei glykosylierten A- und B-Ketten (MLA bzw. MLB). Die A-Kette ist für eine enzymatische Inaktivierung von Ribosomen (Endo et al., 1988) verantwortlich während die B-Kette bei der Carbohydratbindung beteiligt ist. Die beiden Ketten sind durch Disulfidbrücken miteinander verknüpft. Die resultierenden Mistellektin-Monomere können sich unter Ausbildung von nicht kovalenten Bindungen zu Dimeren zusammenlagern.
Mittlerweile ist es möglich, das biologisch aktive Mistellektin auch rekombinant herzustellen. EP 0 751 221 beschreibt die Reindarstellung von Mistellektin-Polypeptiden als strukturell homogene Substanz, wobei ausgehend von den Gensequenzen des Mistellektins rekombinante, hochreine Einzelketten (A-Kette, B-Kette) hergestellt werden, die *in vitro* reassoziiert werden können und so ein rekombinantes Mistellektin-Holoprotein ergeben, das proteinchemisch, enzymatisch und strukturell homogen ist, sogenanntes Aviscumin. Gemäß EP 0751221 ist das rekombinante Mistellektin-Polypeptid sowohl als Holoprotein, als Teilkette und in Form von Subfragmenten für therapeutische Zwecke geeignet und erfindungsgemäß mitumfasst.

Bisher wurden rekombinante Mistellektine insbesondere in der Behandlung von Tumorerkrankungen eingesetzt. In EP 0 751 221 wird zwar erwähnt, dass rekombinante Mistellektine auch für die Behandlung von Infektionserkrankungen denkbar wären, jedoch werden keinerlei Hinweise offenbart, dass eine Behandlung von Virusinfektionen mit rekombinanten Mistellektinen wirksam ist.

Die Aufgabe der vorliegenden Erfindung besteht darin, antivirale Mittel bereitzustellen mit denen Virusinfektionen wirksam behandelt werden können. Eine weitere Aufgabe der vorliegenden Erfindung ist die Bereitstellung eines Arzneimittels sowie pharmazeutischer Zusammensetzungen für die Behandlung von Virusinfektionen.

Die Aufgabe wird durch die Bereitstellung eines antiviralen Mittels samt Bereitstellung eines Arzneimittels sowie einer pharmazeutischen Zusammensetzung gelöst, wobei diese rekombinante Mistellektine zur Behandlung und Prophylaxe von Virusinfektionen enthalten, wobei die rekombinanten Mistellektine die folgenden Aminosäuresequenzen umfassen. Vorzugsweise umfasst das erfindungsgemäße antivirale Mittel die Mistellektin-A-Kette (MLA) beziehungsweise die Mistellektin-B-Kette (MLB), jeweils einzeln oder zusammen, auch in Form von Dimeren (Siehe z.B. EP 0 751 221 oder EP 1 051 495).

Das rekombinante Mistellektin-Polypeptid der Mistellektin-A Kette umfasst die folgenden Sequenzen: SEQ ID No. 1 - 3, einschließlich deren Isoformen oder ein funktionelles Fragment davon.

Das rekombinante Mistellektin-Polypeptid der Mistellektin-B Kette umfasst die folgenden Sequenzen SEQ ID No. 4 - 12, einschließlich deren Isoformen oder ein funktionelles Fragment davon.

### (nachstehend allesamt "rekombinante Mistellektine")

Weiterhin bevorzugt ist Aviscumin eine Heterodimer bestehend aus den Sequenzen SEQ ID No. 1 und SEQ ID No. 4.

Die Erfindung betrifft ein antivirales Mittel enthaltend rekombinantes Mistellektin zur Anwendung bei Virusinfektionen oder um Virusinfektionen vorzubeugen, wobei das rekombinante Mistellektin ausgewählt ist aus der Gruppe der Aminosäuresequenzen SEQ ID No. 1 - 12 oder Teile und Fragmente davon umfasst oder eine Kombination davon.

Der Begriff "funktionelles Fragment" definiert im Zusammenhang mit dieser Erfindung Fragmente der genannten Polypeptide, welche die gleiche biologische Funktion besitzen, wie das mit der jeweiligen Aminosäuresequenz oben dargestellte Polypeptid.

Der Begriff "gleiche biologische Funktion" beschreibt in diesem Zusammenhang beispielsweise, dass Fragmente oder Derivate der Polypeptide die gleichen Signale in einer Zelle induzieren, wie die genannten Peptide. Beispiele für Fragmente sind Peptiddomänen mit definierten Funktionen. Die "gleiche biologische Funktion" umfassen auch die Zytotoxizität, Immunstimmulation (sowohl des nativen, als auch des adaptiven Immunsysterns), Stimulation der Freisetzung von Zytokinen, Antigenität, die Induktion der Expression oder die Aktivierung von Oberflächenmarkern, die Induktion von Apoptose oder Endorphin-Stimulation.

Unter "biologischer Aktivität des rekombinanten Mistellektins" wird hier jede biologische Aktivität aus dem Spektrum der gesamten biologischen Aktivitäten des rekombinanten Mistellektins verstanden. Eine derartige Funktion ist z.B. die pharmakologische Wirkung des rekombinanten Mistellektins.

Untersuchungen von ML-I-Monomeren ergaben 25 verschiedene Isoformen, die auf unterschiedliche Kombinationen verschiedener A- und B-Ketten sowie unterschiedliche Glykosylierungszustände der Ketten zurückzuführen sind.

Die vorliegende Erfindung betrifft daher ebenfalls ein Mistellektin-Polypeptid oder ein Fragment davon, welches die Sequenzvariabilität der verschiedenen MLA- und MLB-Ketten zu den Sequenzen SEQ ID No. 1 - 12 erfindungsgemäß umfasst.

Vorzugsweise enthält das erfindungsgemäße antivirale Mittel ein rekombinantes Mistellektin-Polypeptid mit den Sequenzen SEQ ID No. 1 - 12 oder einem funktionellen Fragment davon oder eine beliebige Kombination davon.

Wie oben bereits erwähnt, werden rekombinante Mistellektine bisher in erster Linie bei der Behandlung von Tumorerkrankungen verwendet. Bis heute ist jedoch nicht aufgezeigt worden, dass rekombinante Mistellektine eine Wirkung auf Virusinfektionen haben. Überraschender Weise hat die Erfindung gezeigt, dass rekombinante Mistellektine wirksam gegen Virusinfektionen eingesetzt werden können, wie in den Beispielen eindrucksvoll gezeigt. Die erfindungsgemäßen Sequenzen, insbesondere Aviscumin, weisen bereits eine Zytotoxizität von 0,5 ng/Plaque auf.

Besonders vorteilhaft weisen die rekombinanten Mistellektine gegenüber den Mistellektinen im Stand der Technik keine Verunreinigungen auf, so dass eine geringere Dosis bereits eine erhöhte Wirksamkeit aufweist. Ferner wird durch rekombinante Mistellektine eine hohe Dosiergenauigkeit erreicht, so dass eine erfolgreiche (lokal)spezifische antivirale Behandlung erlaubt ist.

Das erfindungsgemäße antivirale Mittel wird daher für die Behandlung von einer der folgenden Virusinfektionen verwendet: Herpes-simplex-Virusinfektion, Adenovirusinfektion, Poliovirusinfektion, Pockenvirusinfektion, Parvovirusinfektion, Papovavirusinfektion, Hepadnavirusinfektion, Orthomyovirusinfektion, Papillomavirusinfektion, Paramyxovirusinfektion, Coronavirusinfektion, Picornavirusinfektion, Reovirusinfektion, Togavirusinfektion, Flavivirusinfektion, Arenavirusinfektion, Rhabdovirusinfektion, Retrovirusinfektion.

Weiterhin betrifft die Erfindung die Behandlung von Hautviruswarzen, Anogenitale Warzen, Schleimhautwarzen und maligne Tumore wie Zervixkarzinom, Penis- und Vulvakarzinom, insbesondere im Zuge einer Papillomavirusinfektion (HPV).

Die Erfindung betrifft außerdem ein Arzneimittel für die Behandlung von Virusinfektionen, welches das rekombinante Mistellektin-Polypeptid, gegebenenfalls zusammen mit einem pharmazeutisch verträglichen Träger enthält. Beispiele für besonders geeignete pharmakologisch verträgliche Träger sind dem Fachmann bekannt und umfassen gepufferte Kochsalzlösungen, Wasser, Emulsionen wie z.B. Öl/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden. Die Verabreichung kann lokal, oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden.

Die Erfindung betrifft außerdem eine pharmazeutische Zusammensetzung, welche die erfindungsgemäßen rekombinanten Mistellektin-Polypeptide umfasst. Die Zusammensetzung kann lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen Natriumchlorid-Lösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren).
Die erfindungsgemäße Zusammensetzung kann außerdem Konservierungsmittel und andere Zusätze umfassen, wie z.B. antimikrobielle Verbindungen, Antioxidantien und Komplexbildner. Des Weiteren können Verbindungen wie z.B. Interleukine, Interferone, oder ein unspezifisches immunmodulatorisches Agens enthalten sein. Ebenso können Zytostatika, Antibiotika und Kombinationen davon enthalten sein.

Die pharmazeutische Zusammensetzung wird erfindungsgemäß bei der Behandlung der oben genannten Virusinfektionen verwendet.

Vorzugsweise liegt die erfindungsgemäße Zusammensetzung als Lösung, Gel oder Creme vor.

Die Erfindung betrifft des Weiteren die Verwendung eines rekombinanten Mistellektins zur Herstellung eines antiviralen Mittels zur Behandlung von oben genannten Virusinfektionen. Vorzugsweise umfasst die Behandlung das Verabreichen einer therapeutisch wirksamen Menge des rekombinanten Mistellektins an einen Menschen, welcher an einer Infektion erkrankt ist oder voraussichtlich erkranken wird.

Die Wirksamkeit der Erfindung bei der Behandlung von Virusinfektionen wird beispielhaft anhand der folgenden Untersuchung erläutert.

Nachfolgende Beispiele dienen zur Erläuterung der Erfindung, ohne jedoch die Erfindung auf diese Beispiele zu beschränken.

### Beispiele:

Vero-Zellen (Nierenzellen von African green monkeys) und BGM-Zellen (Nierenzellen von Borgio green monkeys) wurden im Rahmen einer log₁₀ Verdünnungsreihe mit Herpessimplex-Viren vom Typ 1 (HSV-1) für 2 Stunden inkubiert. Nach der Infektion der Zellen wurde das Virus entfernt. Das rekombinante Mistellektin wurde mit Zellkulturmedium verdünnt, das sowohl mit als auch ohne Störsubstanzen verwendet wurde. Als Störsubstanzen wurden 10% fötales Kälberserum und 3% Rinderserumalbumin mit Schaferythrozyten verwendet. Pro well wurden 200 µ1 der Lösung mit rekombinantem Mistellektin auf die infizierten Zellen aufgebracht. Als Viruskontrolle wurde Zellkulturmedium auf die infizierten Zellen aufgebracht. Als Negativkontrolle gegen HSV-1 wurde Aciclovir (1,5 mmol) an Stelle des rekombinanten Mistellektins verwendet. Die Zellen wurden bei 37°C +/-1 °C für 2-7 Tage inkubiert.
Nach der Inkubation wurden die Zellen mit einem inversen Mikroskop auf cytopathische Effekte (CPE) untersucht und es wurde die 50% infektiöse Dosis bezogen auf eine Zellkultur TCID₅₀/ml (Tissue Culture Infectious Dosis) bestimmt. Sofern keine cytopathischen Effekte sichtbar waren, bedeutete dies, dass die Viren erfolgreich durch das rekombinante Mistellektin inaktiviert worden waren.

Um einen detaillierteren Eindruck der Virusreduktion zu erhalten, wurde ein Plaque-Reduktions-Assays durchgeführt, um die antivirale Wirksamkeit des rekombinanten Mistellektins zu bestimmen. Hierfür wurden die Vero-Zellen / BGM-Zellen in 12-well-Platten platziert.
Die semi-adherenten Zellen wurden mit einer log₁₀ Verdünnungsreihe des HSV-1 (100 µl pro well) für 2 Stunden inkubiert. Nach der Infektion der Zellen wurde das Virus entfernt. Das rekombinante Mistellektin wurde mit Zellkulturmedium (mit und ohne Störsubstanzen) verdünnt und mit warmer Agarose 1:1 vermischt. Als Störsubstanzen wurde fötales Kälberserum und Schaferythrozyten verwendet
Von der rekombinanten Mistellektin enthaltenden Lösung wurden 2 ml auf die infizierten Zellen aufgebracht. Als Viruskontrolle wurde Zellkulturmedium auf die infizierten Zellen aufgebracht. Als Negativkontrolle gegen HSV-1 wurde Aciclovir (1,5 mmol) an Stelle des rekombinanten Mistellektins verwendet. Nach der Gelierung der Agarose wurden die Zellen bei 37°C +/- 1 °C für 7 Tage inkubiert. Nach der Inkubationszeit wurden die Zellen für 4 Stunden mit Methanol fixiert, das 4% Natriumchlorid enthielt. Danach wurden die Zellen mit Kristallviolett gefärbt. Die virusinduzierten Plaques wurden unter dem Mikroskop gezählt und der TCID₅₀/ml wurde bestimmt.

Im Rahmen dieser Untersuchung sind die folgenden Validierungsschritte durchgeführt worden:
- Identifizierung des geeigneten Virustiter mit der entsprechenden Zelllinie.
- Identifizierung der Konzentration an rekombinantem Mistellektin, die von der entsprechenden Zelllinie toleriert wird durch Messung der Zytotxizität und Vitalität.
- Identifizierung der Konzentration an rekombinantem Mistellektin, welche die Zelllinie empfänglich für eine Virusinfektion lässt.

### Ergebnisse:

Die Untersuchung zeigte überraschenderweise eine antivirale Wirksamkeit von rekombinantem Mistellektin gegen HSV-1 und gegen Adeno Virus Typ 5. Eine Konzentration von 50 ng/ml von rekombinantem Mistellektin verursachte im Suspensionsassay mit HSV-1 / BGM-Zellen eine Virustiterreduktion von 2,43 log₁₀ ohne Störsubstanzen und ohne die Verwendung von MicroSpin-Säulen (siehe Tabelle 2). Bei einer Konzentration von 500 ng/ml von rekombinantem Mistellektin zeigte sich eine Virustiterreduktion von 2,57 log₁₀ und ≥ 3,29 log₁₀ bei einer Verwendung von MicroSpin-Säulen und FCS als Störsubstanz (siehe Tabelle 2).

Beim Plaque-Reduktions-Assays zeigte sich eine durchschnittliche relative Inhibierung der HSV-1 Infektion von 17,04% bei einer Konzentration von 0,5 ng/ml rekombinante Mistellektin (ohne MicroSpin-Filtration) beziehungsweise eine durchschnittliche relative Inhibierung der HSV-1 Infektion von 7,41% mit MicroSpin-Filtration (siehe Tabelle 3).

Die durchschnittliche relative Inhibierung der Adeno Virus Typ 5 Infektion im Plaque-Reduktions-Assay betrug 79,7 % bei einer Konzentration von 0.5 ng/ml rekombinantem Mistellektin (ohne MicroSpin-Filtration) beziehungsweise 22,2 % mit Microspin-Filtration (siehe Tabelle 4).

**Tabelle 1: Ergebnisse der Zytotoxizitätstests der Testzellen**

| **Zelllinie** | **Vero** | **Vero** | **BGM** | **BGM** |
|---|---|---|---|---|
| | mit microSpin | ohne microSpin | mit microSpin 7 Tage Inkubation | ohne microSpin 7 Tage Inkubation |
| Rekombinantes Mistellektin 5000 ng/ml | zytotoxisch | zytotoxisch | n.t. | n.t. |
| Rekombinantes Mistellektin 500 ng/ml | zytotoxisch | zytotoxisch | zytotoxisch | zytotoxisch |
| Rekombinantes Mistellektin 50 ng/ml | zytotoxisch | zytotoxisch | negativ | zytotoxisch |
| Rekombinantes Mistellektin 5 ng/ml | negativ | zytotoxisch | negativ | negativ |
| Rekombinantes Mistellektin 0,5 ng/ml | negativ | negativ | negativ | negativ |
| Rekombinantes Mistellektin 0,05 ng/ml | negativ | negativ | negativ | negativ |
| Rekombinantes Mistellektin 0,005 ng/ml | n.t. | n.t. | negativ | negativ |
| Negativkontrolle | n.t. | negativ | n.t. | negativ |

| | | | | |
|---|---|---|---|---|
| n.t. = nicht geprüft | | | | |

**Tabelle 2: Test auf antivirale Wirksamkeit von rekombinantem Mistellektin bei HSV-1, Wirt: BGM-Zellen (Zellsuspension)**

| | | HSV-1 Virus (TCID₅₀/ml) | Virustiterreduktion (log₁₀) + 95% conf. limits |
|---|---|---|---|
| **Viruskontrolle** | | 10^{-6,64 +/- 0,46} 10^{-6,93 +/- 0,36} | - |
| **Rekombinantes Mistellektin 5 ng/ml** | oSS, ohne microSpin | 10^{-6,79 +/- 0,36} | Keine Virustiterreduktion |
| | oSS, mit microSpin | 10^{-6,79 +/- 0,36} | Keine Virustiterreduktion |
| | FCS, ohne microSpin | 10^{-6,79 +/- 0,54} | Keine Virustiterreduktion |
| | FCS, mit microSpin | 10^{-6,64 +/- 0,46} | Keine Virustiterreduktion |
| **Rekombinantes Mistellektin 50 ng/ml** | oSS, ohne microSpin | 10^{-4,21 +/- 0,54} | 2,43 +/- 0,71 |
| | oSS, mit microSpin | 10^{-6,36 +/- 0,56} | 0,28 +/- 0,72 |
| | FCS, ohne microSpin | 10^{-6,36 +/- 0,54} | 0,28 +/- 0,65 |
| | FCS, mit microSpin | 10^{-6,36 +/- 0,46} | 0,28 +/- 0,65 |
| **Rekombinantes Mistellektin 500 ng/ml** | FCS, mit microSpin | 10^{-4,07 +/- 0,49} | 2,57 +/- 0,67 |
| | | ≤10^{-3,64 +/- 0,28} | ≥ 3,29 +/- 0,54 |

| | | | |
|---|---|---|---|
| oSS= ohne Störsubstanzen | | | |

**Tabelle 3: Test auf antivirale Wirksamkeit von rekombinantem Mistellektin, Plaque-Assay mit HSV-1 auf Vero-Zellen**

| Virusverdünnung | | Viruskontrolle | Aciclovir (1,5mmol) | Rekombinates Mistellektin | | |
|---|---|---|---|---|---|---|
| | | | | 0,5 ng/ml ohne Spin | 0,5 ng/ml mit Spin | 0,5 ng/ml mit Spin |
| | Anzahl Plaques | > 50, > 50, 6 | 0, 0, 0 | > 50, > 50, >50 | 10, > 50, > 50 | zytotoxisch |
| | Mittelwert | > 35 | 0 | > 50 | > 36 | |
| 10^{-5,0} | StabW | n.d. | 0 | n.d. | n.d. | n.d. |
| | Relative Infektiosität | 100 | 0 | 100 | 100 | n.d. |
| | Relative Hemmung | 0 | 100 | 0 | 0 | n.d. |
| | Anzahl Plaques | 5,5,4,4 | 0, 0, 0, 0 | 6,5,3,2 | 3, 4, 4, 3 | zytotoxisch |
| | Mittelwert | 4,5 | 0 | 4,0 | 3,5 | |
| 10^{-5,48} | StabW | 0,57 | 0 | 1,82 | 0,57 | n.d. |
| | Relative Infektiosität | 100 | 0 | 88,88 | 77,77 | n.d. |
| | Relative Hemmung | 0 | 100 | 11,12 | 22,23 | n.d. |
| | Anzahl Plaques | 2,3,3,2 | 0, 0, 0, 0 | 1, 2, 1, 2 | 2, 3, 1, 4 | zytotoxisch |
| | Mittelwert | 2,5 | 0 | 1,5 | 2,5 | |
| 10^{-5,95} | StabW | 0,57 | 0 | 0,57 | 1,29 | n.d. |
| | Relative Infektiosität | 100 | 0 | 60,0 | 100 | n.d. |
| | Relative Hemmung | 0 | 100 | 40,0 | 0 | n.d. |
| **Durchschnittliche relative Hemmung** | | n.a | **100** | **17,04** | **7,41** | n.d. |

| | | | | | | |
|---|---|---|---|---|---|---|
| n.d. = nicht detektierbar n.a. = nicht zu bewerten | | | | | | |

**Tabelle 4: Test auf antivirale Wirksamkeit von rekombinantem Mistellektin, Plaque-Assay mit Adeno Virus Typ 5 auf BGM-Zellen**

| Virusverdünnung | | Viruskontrolle | Rekombinates Mistellektin | | |
|---|---|---|---|---|---|
| | | | 0,5 ng/ml ohne Spin | 0,5 ng/ml mit Spin | 0,5 ng/ml mit Spin |
| | Anzahl Plaques | 5, 5, 8 | 2, 3, 0 | 11, 8, 8 | zytotoxisch |
| | Mittelwert | 6 | 1,66 | 9 | |
| 10^{-4,0} | StabW | 1,73 | 1,52 | 1,73 | n.d. |
| | Relative Infektiosität | 100 | 27,7 | 100 | n.d. |
| | Relative Hemmung | 0 | 72,3 | 0 | n.d. |
| | Anzahl Plaques | 3, 4, 2, 3 | 0, 0, 0, 0 | 4, 2, 2, 4 | zytotoxisch |
| | Mittelwert | 3 | 0 | 3 | |
| 10^{-4,48} | StabW | 0,81 | 0 | 1,15 | n.d. |
| | Relative Infektiosität | 100 | 0 | 100 | n.d. |
| | Relative Hemmung | 0 | 100 | 0 | n.d. |
| | Anzahl Plaques | 1, 1, 2, 2 | 0, 0, 2, 0 | 0,0,0,2 | zytotoxisch |
| | Mittelwert | 1,5 | 0,5 | 0,5 | |
| 10^{-4,95} | StabW | 0,57 | 1,0 | 1,0 | n.d. |
| | Relative Infektiosität | 100 | 33,3 | 33,3 | n.d. |
| | Relative Hemmung | 0 | 66,7 | 66,7 | n.d. |
| **Durchschnittliche relative Hemmung** | | n.a | **79,7** | **22,2** | n.d. |

| | | | | | |
|---|---|---|---|---|---|
| n.d. = nicht detektierbar n.a. = nicht zu bewerten | | | | | |

## Patentansprüche

1. Antivirales Mittel oder Arzneimittel enthaltend rekombinantes Mistellektin zur Verwendung in der Behandlung von Virusinfektionen oder um Virusinfektionen vorzubeugen, wobei das rekombinante Mistellektin ausgewählt ist aus der Gruppe der Aminosäuresequenzen SEQ ID No. 1 - 12 oder funktionelle Teile und Fragmente davon umfasst oder eine Kombination davon.

2. Antivirales Mittel oder Arzneimittel zur Verwendung nach Anspruch 1, wobei das rekombinante Mistellektin-Polypeptid eine Mistellektin-A-Kette ist, ausgewählt aus der Gruppe der Aminosäuresequenzen SEQ ID No. 1 - 3 oder funktionelle Teile und Fragmente davon umfasst oder eine Kombination davon.

3. Antivirales Mittel oder Arzneimittel zur Verwendung nach Anspruch 1, wobei das rekombinante Mistellektin-Polypeptid eine Mistellektin-B-Kette ist, ausgewählt aus der Gruppe der Aminosäuresequenzen SEQ ID No. 4 - 12 oder funktionelle Teile und Fragmente davon umfasst oder eine Kombination davon.

4. Antivirales Mittel oder Arzneimittel zur Verwendung nach einem der Ansprüche 1 bis 3 zur Verwendung bei einer der folgenden Virusinfektionen: Herpes-simplex-Virusinfektion, Adenovirusinfektion, Poliovirusinfektion, Pockenvirusinfektion, Parvovirusinfektion, Papovavirusinfektion, Hepadnavirusinfektion, Orthomyovirusinfektion, Papillomavirusinfektion, Paramyxovirusinfektion, Coronavirusinfektion, Picornavirusinfektion, Reovirusinfektion, Togavirusinfektion, Flavivirusinfektion, Arenavirusinfektion, Rhabdovirusinfektion, Retrovirusinfektion.

5. Antivirales Mittel oder Arzneimittel zur Verwendung nach einem der Ansprüche 1 bis 4 zur Verwendung bei Hautviruswarzen, Anogenitale Warzen, Schleimhautwarzen und maligne Tumore wie Zervixkarzinom, Penis- und Vulvakarzinom.

6. Antivirales Mittel oder Arzneimittel zur Verwendung gemäß einem der Ansprüche 1 bis 5 enthaltend einen pharmazeutisch verträglichen Träger oder ggfs. weiteren Hilfs- und Zusatzstoffen.

7. Antivirales Mittel oder Arzneimittel zur Verwendung gemäß einem der Ansprüche 1 bis 6 enthaltend eine pharmazeutische Zusammensetzung, wobei die Zusammensetzung in Form einer Lösung, Gel oder Creme vorliegt.

## Claims

1. An antiviral agent or medicinal drug' comprising recombinant mistletoe lectin for use in the treatment of virus infections or so as to prevent virus infections, wherein the recombinant mistletoe lectin is selected from the group of the amino acid sequences SEQ ID Nos. 1 - 12, or comprises functional parts and fragments thereof, or a combination thereof.

2. The antiviral agent or medicinal drug for use according to claim 1, wherein the recombinant mistletoe lectin polypeptide is a mistletoe lectin A-chain, selected from the group of the amino acid sequences SEQ ID Nos. 1 - 3, or comprises functional parts and fragments thereof, or a combination thereof.

3. The antiviral agent or medicinal drug for use according to claim 1 wherein the recombinant mistletoe lectin polypeptide is a mistletoe lectin B-chain, selected from the group of the amino acid sequences SEQ ID Nos. 4 - 12, or comprises functional parts and fragments thereof, or a combination thereof.

4. An antiviral agent or medicinal drug for use according to any one of claims 1 to 3 for use with one of the following virus infections: herpes simplex virus infection, adenovirus infection, poliovirus infection, poxvirus infection, parvovirus infection, papovavirus infection, hepadnavirus infection, orthomyxovirus infection, papilloma virus infection, paramyxovirus infection, coronavirus infection, picornavirus infection, reovirus infection, togavirus infection, flavivirus infection, arenavirus infection, rhabdovirus infection, and retrovirus infection.

5. An antiviral agent or medicinal drug for use according to any one of claims 1 to 4 for use with skin virus warts, anogential warts, mucous membrane warts and malignant tumors such as cervical cancer, penis and vulvar cancer.

6. An antiviral agent or medicinal drug for use according to any one of claims 1 to 5 comprising a pharmaceutically compatible carrier or if applicable further auxiliaries and additives.

7. An antiviral agent or medicinal drug for use according to any one of claims 1 to 6 comprising a pharmaceutical composition, wherein the composition is present in the form of a solution, gel or cream.

## Revendications

1. Agent antiviral ou médicament comprenant de la lectine de gui recombinante pour une utilisation dans le traitement d'infections virales ou afin de prévenir des infections virales, où la lectine de gui recombinante est choisie dans le groupe des séquences d'acides aminés SEQ ID N ° 1 à 12, ou comprend des parties fonctionnelles et des fragments de celles-ci, ou une combinaison de celles-ci.

2. Agent antiviral ou médicament pour une utilisation selon la revendication 1, dans lequel le polypeptide de la lectine de gui recombinante est une chaîne A de lectine de gui, choisie dans le groupe des séquences d'acides aminés SEQ ID n° 1 à 3, ou comprend des parties fonctionnelles et des fragments de celles-ci, ou une combinaison de celles-ci.

3. Agent antiviral ou médicament pour une utilisation selon la revendication 1, dans lequel le polypeptide de la lectine de gui recombinante est une chaîne B de lectine de gui, choisie dans le groupe des séquences d'acides aminés SEQ ID n° 4 à 12, ou comprend des parties fonctionnelles et des fragments de celles-ci, ou une combinaison de celles-ci.

4. Agent antiviral ou médicament pour une utilisation selon l'une quelconque des revendications 1 à 3, pour une utilisation dans le cas de l'une des infections virales suivantes : infection par le virus de l'herpès simplex, infection par adénovirus, infection par poliovirus, infection par poxvirus, infection par parvovirus, infection par papovavirus, infection par hépadnavirus, infection par orthomyxovirus, infection par papillomavirus, infection par paramyxovirus, infection par coronavirus, infection par picornavirus, infection par réovirus, infection par togavirus, infection par flavivirus, infection par arénavirus, infection par rhabdovirus, et infection par rétrovirus.

5. Agent antiviral ou médicament pour une utilisation selon l'une quelconque des revendications 1 à 4, pour une utilisation contre les verrues cutanées d'origine virale, les verrues anogénitales, les verrues de la membrane muqueuse et les tumeurs malignes telles que le cancer du col de l'utérus, les cancers de la vulve et du pénis.

6. Agent antiviral ou médicament pour une utilisation selon l'une quelconque des revendications 1 à 5, comprenant un vecteur pharmaceutiquement compatible ou, le cas échéant, des auxiliaires et des additifs.

7. Agent antiviral ou médicament pour une utilisation selon l'une quelconque des revendications 1 à 6, comprenant une composition pharmaceutique, où la composition est présente sous la forme d'une solution, d'un gel ou d'une crème.
